# EUROPEAN PATENT APPLICATION

(11) **EP 1 216 697 A2**
(43) Date of publication of application: **26.06.2002**
(21) Application number: 01600025.9
(22) Date of filing: 27.11.2001
(51) Int. Cl.: A61K 7/48, C11D 9/18

(54) **Mixture of vulcanic soil from Thira island and soap, use as cleansing and cosmetic for hands and nails, and method of preparation**

(30) Priority: 08.12.2000 GR 2000100423
(71) Applicant: Papadakos, Ioannis, 154 52 P. Psychiko Attikis (GR)
(72) Inventor: Papadakos, Ioannis, 154 52 P. Psychiko Attikis (GR)

(57) **Abstract**

The Mixture prepared with Thira volcanic soil [pumice-stone] and soap, is consisting of grains of Thira pumice-stone mixed with soap flakes, in various proportions accord ing to its application, as it can effectively be used by a wide list of professionals such as painters,people working in garages and vehicle services,butchers,medicine doctors etc, as a cleansing of hands,nails and the area around nails,and it also acts as disinfective,re-moving the dead cells of the hands skin and the small peels around the nails and/or any germs eventually created.

## Description

The present invention refers to a mixture consisting of grains [fragments] of vulcanic soil from Thira island and flakes of soap,which mixture can be used widely for the cleansing,the disinfection and cure of the hands and the area around the nails,which mixture was invented,prepared and is being distributed to the market for the first time,absolutely and solely by only me.

As known,in certain works such as of the painter,of the people working in garages and vehicle reparation stations etc., the said workers' hands and especially the area around the nails become dirty from the substances and other materials used by them during their work. Also,in the case of butchers,by their continuous touching of the meat,germs are created in their hands and nails,and for this reason is required a pedantic cleansing as well as disinfection of the hands. Same also applies to the medicine doctors who are obligated to have a continuous and pedantic cleansing and disinfection of their hands,many times during their work.

By this invention is produced a mixture of wide application,which can be used by a wide list of professions requiring a fine, easy and quick cleansing of hands and nails, as the its synthesis can be modified as to give a desired result of cleansing depending on the level of hands dirtiness, while it is disinfective rejecting the dead cells of the hands skin,and the small peels in the area around the nails,without the use of special tools.

The preparation of the mixture of cleansing,cure and disinfection of hands and nails in general,is based on mixing of grains [fragments] of vulcanic soil from Thira island,commonly known as pumice-stone,with flakes of soap containing much olive oil or glycerine,which have the quality for the fine and hygienic cleansing and for the simultaneous cure of the skin.

Some suggested syntheses are prepared with Thira pumice-stone of grains of 0-0-2 mm and soap flakes of olive oil ,in accordance with the following proportions
1. One weight portion of Thira pumice-stone against one weight portion of soap flakes
2. One weight portion of Thira pumice-stone against two weight portions of soap flakes
3. One weight portion of Thira pumice-stone against three weight portions of soap flakes, and
4. One weight portion of Thira pumice-stone against four weight portions of soap flakes.

The use of the prepared mixture is the same as in the case of soap,while in the most difficult areas, as in the area around the nails where much more dirt is concentrated,some sort of rubbing may be applied,so that,fine cleansing is achieved. The result of the use of the prepared mixture is amazing,since all dirt is perfactly removed from the hands and nails and the area around the nails is simultaneously cleared of undesirable and bothering small peels and/or germs, while the skin remains soft and smooth.

The soap flakes come from soap of virgin olive oil, being easily found in the market,which soap of virgin olive oil is hygienic and cleaning perfectly and also acts as a cosmetic of hands,this not necessarily means that can not be utilised soap flakes of any other synthesis suitable for the this specific scope,such as glycerine soap or even powder of washing machines, etc.

Moreover,the mm of the grains of the Thira pumice-stone of the above syntheses may be modified.

Finally,the synthesis of the invention may also be utilised for the cleansing and cure of other areas of the human body,such as feet, foot nails,heels,knees,elbows,even the face,always with the same spectacular results.

## Claims

1. Mixture prepared from Thira pumice-stone and soap, as a cleansing and cosmetic for hands and nails, **characterised by** its preparation from grains of Thira pumice-stone and soap flakes.

2. Mixture prepared from Thira pumice-stone and soap,as a cleansing and cosmetic for hands and nails,as per claim 1 above,**characterised by** the fact that the Thira pumice stone has a grain dimension 0-0-2 mm and the soap flakes come from soap of virgin olive oil,being easily found in the market.

3. Mixture prepared from Thira pumice-stone and soap,as cleansing and cosmetic for hands and nails,as per claims under 1 and 2 above,**characterised by** the fact that it can be prepared in accordance with the following proportions
1. One weight portion of Thira pumice-stone against one weight portion of soap flakes
2. One weight portion of Thira pumice-stone against two weight portions of soap flakes
3. One weight portion of Thira pumice-stone against three weight portions of soap flakes, and
4. One weight portion of Thira pumice-stone against four weight portions of soap flakes.

4. Mixture prepared with Thira pumice-stone and soap,as a cleansing and cosmetic of hands and nails,as per claims 1,2 and 3 above, **characterised by** the fact that it also acts as disinfective since it removes the dead cells of the skin,the small peels of the area around the nails and/or the germs which may possibly have been created.

5. Mixture prepared with Thira pumice-stone and soap,as a cleansing and cosmetic of hands and nails,as per claims 1,2,3 and 4 above, **characterised by** the fact that it can be used by a wide list of professionals ,such as painters ,people working in garages and vehicles services,butchers,medicine doctors etc., by women for kind treatment of hands and nails,as well as other areas of the human body ,such as elbows,feet,foot nails,knees etc.

6. Mixture prepared with Thira pumice-stone and soap,as a cleansing and cosmetic, as per claims 1,2,3,4 and 5 above, **characterised by** the fact that it can also be prepared by pumice-stone of any grain dimensions or flakes of any soap.

7. Method of preparation of synthesis of Thira pumice-stone and soap,**characterised by** the fact that it is based on the mixture of Thira pumice-stone of a grain dimension of 0-0-2 mm with flakes of pure soap from olive oil of the market,in various proportions according to the application of the mixture.
